# EUROPÄISCHE PATENTSCHRIFT

(11) **EP 1 222 911 B1**
(45) Veröffentlichungstag und Bekanntmachung des Hinweises auf die Patenterteilung: **25.10.2006**
(21) Anmeldenummer: 01130827.7
(22) Anmeldetag: 27.12.2001
(51) Int. Cl.: A61K 8/49, A61Q 5/10

(54) **Haarfärbemittel und Verfahren zum Färben von menschlichen Haaren**
Hair dye composition and process for dyeing human hair
Colorant pour cheveux et procédé pour la coloration des cheveux naturels

(30) Priorität: 04.01.2001 DE 10100272; 04.01.2001 DE 10100271; 04.01.2001 DE 10100269; 04.01.2001 DE 10100268; 27.01.2001 DE 10103625; 27.01.2001 DE 10103626
(43) Veröffentlichungstag der Anmeldung: 17.07.2002
(73) Patentinhaber: KPSS-Kao Professional Salon Services GmbH, 64297 Darmstadt (DE)
(72) Erfinder: Lorenz, H., 64401 Gross Bieberau (DE); Wagner, H.R., 64291 Darmstadt (DE); Garbe, B., 64625 Bensheim (DE); Kaffenberger, K., 64665 Alsbach-Hähnlein (DE)

(56) Entgegenhaltungen:
- EP-A- 0 376 078
- EP-A- 0 467 026
- DE-A- 2 359 399
- DE-A- 2 447 017
- DE-A- 4 445 282
- DE-A- 19 859 682
- DE-A- 19 859 800
- US-A- 4 003 699
- US-A- 4 129 413

## Beschreibung

Die Erfindung betrifft ein Haarfärbemittel auf Basis von Oxidationsfarbstoff-Vorprodukten, das nach dem Vermischen mit einer Peroxid-Zusammensetzung auf das menschliche Haar aufgebracht wird, und ein Verfahren zur Haarfärbung unter Verwendung dieses Mittels.

Der die Oxidationsfarbstoffvorprodukte enthaltenden Zusammensetzung wird grundsätzlich ein Reduktionsmittel, vorzugsweise Ascorbinsäure oder ein Alkalisulfit, zugesetzt, um diese Vorprodukte gegen eine unerwünschte vorzeitige Oxidation zu stabilisieren (vgl. hierzu beispielsweise die Monographie von K. Schrader, Grundlagen und Rezepturen der Kosmetika, 2. Aufl. (1989), S. 786).

Diese seit Jahrzehnten geübte Praxis weist jedoch den Nachteil auf, daß mit weiteren Rezepturbestandteilen Unverträglichkeiten auftreten können. Darüberhinaus ist bei der Applikation der anwendungsfertigen Färbemischung, d.h. bei der Mischung der Oxidationsfarbstoffvorprodukt-Zusammerisetzung, die in der Regel eine wäßrige Lösung, Emulsion, Dispersion oder Gel darstellt, mit der Peroxid-Zusammensetzung ein erheblicher Peroxid-Überschuß erforderlich, um die antioxidierende Wirkung dieser Reduktionsmittel aufzuheben. Gleichwohl kann die Färbereaktion verzögert sein, was sich auf die Qualität der erhaltenden Färbung, insbesondere deren Intensität, auswirken kann.
EP-A2-376078 offenbart Haarfärbemittel auf Basis von Oxidationsfarbstoff-Vorprodukten, Di- und Tetra-Aminopyrimidine und direktziehende Nitro Farbstoffe, die auch Reduktionsmittel enthalten können. Außerdem dauert der Färbevorgang verhältnismäßig lange.
Es wurde nunmehr überraschenderweise gefunden, daß sich dieses Problem dadurch lösen läßt und auf die Anwesenheit eines Reduktionsmittels verzichtet werden kann, wenn man als Oxidationsfarbstoffvorprodukte eine Kombination aus mindestens einem Tetraaminopyrimidin, insbesondere 2,4,5,6-Tetraaminopyrimidin, und/oder einem Hydroxytriaminopyrimidin, insbesondere 4-Hydroxy-2,5,6-triaminopyrimidin bzw. deren Salzen, und mindestens einer Kupplersubstanz, ausgewählt aus Resorcin, 2-Methylresorcin, 4-Chlorresorcin, 2-Aminophenol, 3-Amino-phenol, 5-Amino-2-methylphenol, 6-Amino-2-methylphenol, α-Naphthol, 1,3-Di-aminobenzol, 2-Amino-4-hydroxyethylaminoanisol und/oder 2,4-Diaminophenoxyethanol bzw. deren Salzen, sowie noch mindestens einen kationischen direktziehenden Farbstoff einsetzt.

Bei Anwendung dieser Gemische mit Peroxiden, insbesondere Wasserstoffperoxid, werden nach kurzer Einwirkungszeit ausdrucksvolle, intensive Färbungen im Rotsowie im Braun-, Blau- und Violettbereich erhalten, die durch Zusatz weiterer Kupplersubstanzen auch zu anderen Nuancen variierbar sind.

Die eingesetzten Tetraaminopyrimidine sind ebenso wie die Hydroxytriaminopyrimidine als Entwicklersubstanzen in Haarfärbemitteln an sich bekannt, letztere z.B. aus der EP-B 467 026, und bedürfen keiner näheren Erläuterung.

Sie können als freie Basen oder, sofern aus Löslichkeitsgründen erforderlich, auch als wasserlösliche Salze, insbesondere als Hydrochloride oder Sulfate, eingesetzt werden.

Die erfindungsgemäßen Haarfärbemittel können, neben den obengenannten essentiellen Bestandteilen, weitere Oxidationsfarbstoffvorprodukte enthalten.
Beispiele hierfür sind 1-Methoxy-2-amino-4-(β-hydroxyethylamino)benzol, 2-Amino-3-hydroxypyridin, 2,5-Diaminopyridin, 3-Amino-2-methylamino-6-methoxypyridin, 2-(Dimethylamino)-5-aminopyridin, 2-Amino-N,N-diethylaminotoluol, 2-Amino-4-chlorphenol, 1,6-Dihydroxynaphthalin, 1,7-Dihydroxynaphthalin, 1-Methyl-2-hydroxy-4-aminobenzol, 5-Amino-2-methoxyphenol, 2-Methyl-5-hydroxyethylaminophenol, 4-Amino-3-methylphenol, bzw. deren wasserlöslichen Salze.

Damit soll jedoch der Zusatz weiterer Entwickler- und Kupplersubstanzen keineswegs ausgeschlossen sein.

Bei Anwendung dieser Zusammensetzungen auf Basis einer üblichen Grundlage werden, wie bereits erwähnt, nach der Oxidation mit Peroxid auch schon nach relativ kurzer Einwirkungszeit sehr ausdrucksvolle, intensive, dauerhafte Haarfärbungen vor allem im Rotbereich erhalten, die durch Zusatz entsprechender weiterer Kupplersubstanzen noch zu anderen Farbnuancen variiert werden können

Die Gesamtkonzentration der Entwicklersubstanzen liegt üblicherweise zwischen etwa 0,05 und 5%, vorzugsweise 0,1 und 4%, insbesondere 0,25 bis 0,5% und 2,5 bis 3 Gew.-% der Gesamtzusammensetzung des Haarfärbemittels (ohne Oxidationsmittel), wobei sich die Angaben jeweils auf den Anteil an freier Base beziehen.

Das bevorzugte Gewichtsverhältnis von Entwickler- zu Kupplersubstanzen liegt dabei zwischen etwa 1 : 8 bis 8 : 1, vorzugsweise etwa 1 : 5 bis 5 : 1, insbesondere 1 : 2 bis 2 : 1.

Die Kupplersubstanz(en) als Reaktionspartner der Entwicklersubstanz(en) liegen in den erfindungsgemäßen Haarfärbemitteln etwa im gleichen molaren Anteil wie die Entwicklersubstanzen vor, d. h., also in Mengen von 0,05 bis 5,0 %, vorzugsweise 0,1 bis 4 %, insbesondere 0,5 bis 3 Gew.-% der Gesamtzusammensetzung (ohne Oxidationsmittel), wobei sich die Angaben jeweils auf den Anteil an freier Base beziehen.

Der Anteil der direktziehenden Farbstoffe in den erfindungsgemäßen Zusammensetzungen ist variabel und liegt zwischen etwa 0,005 bis etwa 5, vorzugsweise 0,01 bis 2,5, insbesondere 0,1 bis 1 Gew.-% des Mittels.

Als direktziehende Haarfarbstoffe können im Prinzip alle für diesen Zweck vorgeschlagenen kationischen Farbstoffe verwendet werden.

Bevorzugt sind die sogenannten "Arianor"-Farbstoffe; vgl. K. Schrader, "Grundlagen und Rezepturen der Kosmetika", 2.Aufl. (1989), S.811.
Der Einsatz derselben in konventionellen Oxidationsfarbstoff-Zusammensetzungen, die Reduktionsmittel enthielten, war bisher aufgrund fehlender Kompatibilität nicht möglich.

Besonders geeignete basische (kationische) Farbstoffe sind:

| | |
|---|---|
| Basic Blue 6, | C.I.-No. 51,175; |
| Basic Blue 7, | C.I.-No. 42,595; |
| Basic Blue 9, | C.I.-No. 52,015; |
| Basic Blue 26, | C.I.-No. 44,045; |
| Basic Blue 41, | C.I.-No. 11,154; |
| Basic Blue 99, | C.I.-No. 56,059; |
| Basic Brown 4, | C.I.-No. 21,010; |
| Basic Brown 16, | C.I.-No. 12,250; |
| Basic Brown 17, | C.I.-No. 12,251; |
| Natural Brown 7, | C.I.-No. 75,500; |
| Basic Green 1, | C.I.-No. 42,040; |
| Basic Red 2, | C.I.-No. 50,240; |
| Basic Red 12, | C.I.-No. 48,070; |
| Basic Red 22, | C.I.-No. 11,055; |
| Basic Red 51, | |
| Basic Red 76, | C.I.-No. 12,245; |
| Basic Violet 1, | C.I.-No. 42,535; |
| Basic Violet 3, | C.I.-No. 42,555; |
| Basic Violet 10, | C.I.-No. 45,170; |
| Basic Violet 14, | C.I.-No. 42,510; |
| Basic Yellow 57, | C.I.-No. 12,719. |
| Basic Yellow 87 und | |
| Basic Orange 31. | |

Selbstverständlich ist auch die Verwendung entsprechender direktziehender Pflanzenfarbstoffe möglich.

Die erfindungsgemäßen Haarfärbemittel können die in solchen Mitteln üblichen Grund- und Zusatzstoffe, ausgenommen Reduktionsmittel wie Konditioniermittel, etc. enthalten, die dem Fachmann aus dem Stand der Technik bekannt und beispielsweise in der Monographie von K. Schrader, "Grundlagen und Rezepturen der Kopsmetika", 2. Aufl. (Hüthig Buch Verlag, Heidelberg, 1989), S. 782 bis 815, beschrieben sind.
Sie können als Lösungen, Cremes, Gele oder auch in Form von Aerosol-Präparaten vorliegen; geeignete Trägermaterial-Zusammensetzungen sind aus dem Stand der Technik hinreichend bekannt.
Zur Applikation wird das erfindungsgemäße Oxidationsfarbstoff-Vorprodukt mit einem Oxidationsmittel vermischt. Bevorzugtes Oxidationsmittel ist Wasserstoffperoxid, beispielsweise in 2- bis 6-prozentiger Konzentration.
Es können jedoch auch andere Peroxide wie Harnstoffperoxid und Melaminperoxid eingesetzt werden.
Der pH-Wert des applikationsfertigen Haarfärbemittels, d. h. nach Vermischung mit Peroxid, kann sowohl im schwach sauren, d. h. einem Bereich von 5,5 bis 6,9, im neutralen als auch im alkalischen Bereich, d. h. zwischen pH 7,1 und 10 liegen.

Die folgenden Beispiele dienen der Illustration der Erfindung.

**Grundlage**

| | |
|---|---|
| Stearylalkohol | 8,0 (Gew.-%) |
| Kokosfettsäuremonoethanolamid | 4,5 |
| 1,2-Propandiolmono/distearat | 1,3 |
| Kokosfettalkoholpolyglykolether | 4,0 |
| Natriumlaurylsulfat | 1,0 |
| Ölsäure | 2,0 |
| 1,2-Propandiol | 1,5 |
| Na-EDTA | 0,5 |
| Eiweißhydrolysat | 0,5 |
| Parfum | 0,4 |
| Ammoniak, 25%ig | 8,5 |
| Ammoniumchlorid | 0,5 |
| Panthenol | 0,8 |
| Wasser | ad 100,0 |

Die erfindungsgemäße Entwickler-Kuppler-Kombination wurde, jeweils unter entsprechender Verringerung des Wassergehalts, in diese Grundlage eingearbeitet.

Die Ausfärbungen erfolgen jeweils an Woll-Läppchen und Strähnen aus gebleichtem Menschenhaar durch Aufbringung einer einen alkalischen pH-Wert aufweisenden Mischung aus Farbstoff-Vorprodukt und 6%-iger Wasserstoffperoxid-Lösung und zwanzigminütiger Einwirkung bei Zimmertemperatur, Auswaschen und Trocknen.

### Beispiele (in Gew.-%)

| Nr. | 1 | 2 | 3 | 4 | 5 | 6 |
|---|---|---|---|---|---|---|
| 2,4,5,6-Tetraaminopyrimidinsulfat | -- | -- | -- | -- | -- | -- |
| 4-Hydroxy-2,5,6-triaminopyrimidinsulfat | 2,20 | 2,20 | 1,50 | 1,50 | 2,20 | 2,20 |
| Resorcin | -- | -- | -- | -- | -- | -- |
| 2-Methylresorcin | 1,10 | 1,10 | 0,75 | 0,75 | -- | -- |
| 4-Chlorresorcin | -- | -- | -- | -- | 1,30 | 1,30 |
| Basic Red 76 | 0,70 | 0,70 | -- | -- | 0,50 | 0,50 |
| Basic Red 51 | -- | -- | 0,10 | 0,10 | 0,05 | 0,05 |
| Natriumsulfit | -- | 1,00 | -- | 1,00 | -- | 1,00 |

In allen Fällen wurden glänzende, kräftige rote Färbungen erhalten, wobei überraschenderweise die erfindungsgemäßen Zusammensetzungen Nr. 1,3, und 5 durchgängig eine stärkere Farbintensität sowie einen ausgeprägteren Glanz aufwiesen.
Auch erfolgte der Farbaufzug schneller (bereits nach etwa 15 Minuten) als mit den Zusammensetzungen, die ein Reduktionsmittel enthielten.
Nach dreimonatiger Lagerung bei 40°C traten bei den genannten Beispielen keinerlei Veränderungen auf, während die Zusammensetzungen nach den Beispielen 2, 4 und 6 für die Haarfärbung nicht mehr geeignet waren.

### Beispiele (in Gew.-%)

| Nr. | 7 | 8 |
|---|---|---|
| 2,4,5,6-Tetraaminopyrimidinsulfat | 1,10 | 1,10 |
| 4-Hydroxy-2,5,6-triaminopyrimidinsulfat | -- | -- |
| 1-Naphthol | -- | -- |
| 3-Aminophenol | 0,50 | 0,50 |
| 2-Methyl-5-aminophenol | -- | -- |
| Basic Red 76 | -- | -- |
| Basic Red 51 | 0,10 | 0,10 |
| Natriumsulfit | -- | 1,00 |
| Färbung | Rotbraun | |

### Beispiele (in Gew.-%)

| Nr. | 9 | 10 | 11 | 12 |
|---|---|---|---|---|
| 2,4,5,6-Tefraaminopyrimidinsulfat | -- | -- | -- | -- |
| 4-Hydroxy-2,5,6-triaminopyrimidinsulfat | 1,10 | 1,10 | 1,10 | 1,10 |
| 1-Naphthol | -- | -- | -- | -- |
| 3-Aminophenol | 0,50 | 0,50 | -- | -- |
| 2-Methyl-5-aminophenol | -- | -- | 0,70 | 0,70 |
| Basic Red 76 | 0,50 | 0,50 | 0,50 | 0,50 |
| Basic Red 51 | -- | -- | -- | -- |
| Natriumsulfit | -- | 1,00 | -- | 1,00 |
| Färbung | Rotviolett | | violettstichiges Rot | |

In allen Fällen wurden glänzende, kräftige Färbungen erhalten, wobei überraschenderweise die erfindungsgemäßen Zusammensetzungen Nr. 7, 9 und 11 durchgängig eine stärkere Farbintensität sowie einen ausgeprägteren Glanz aufwiesen.

Auch erfolgte der Farbaufzug schneller (bereits nach etwa 15 Minuten) als mit den Zusammensetzungen, die ein Reduktionsmittel enthielten.
Nach dreimonatiger Lagerung bei 40°C traten bei den genannten Beispielen keinerlei Veränderungen auf, während die Zusammensetzungen nach den Beispielen 8, 10 und 12 für die Haarfärbung nicht mehr geeignet waren.

### Beispiele (in Gew.-%)

| Nr. | 13 | 14 | 15 | 16 | 17 | 18 | 19 | 20 | 21 | 22 |
|---|---|---|---|---|---|---|---|---|---|---|
| 2,4,5,6-Tetraaminopyrimidinsulfat | 1,08 | 1,08 | 1,08 | 1,08 | 1,08 | 1,08 | -- | -- | -- | -- |
| 4-Hydroxy-2,5,6-triaminopyrimidinsulfat | -- | -- | -- | -- | -- | -- | 1,08 | 1,08 | 1,08 | 1,08 |
| 1,3-Diaminobenzol | -- | -- | 0,50 | 0,50 | -- | -- | 0,50 | 0,50 | -- | -- |
| 2-Amino-4-hydroxyethylaminoanisolsulfat | 1,25 | 1,25 | -- | -- | -- | -- | -- | -- | -- | -- |
| 2,4-Diaminophenoxyethanolhydrochlorid | -- | -- | -- | -- | 1,10 | 1,10 | -- | -- | 1,10 | 1,10 |
| Basic Brown 16 | -- | -- | -- | -- | -- | -- | 0,50 | 0,50 | -- | -- |
| Basic Red 51 | 0,15 | 0,15 | -- | -- | -- | -- | -- | -- | -- | -- |
| Basic Yellow 57 | -- | -- | -- | -- | 0,50 | 0,50 | -- | -- | -- | -- |
| Basic Blue 99 | -- | -- | 0,30 | 0,30 | -- | -- | -- | -- | 0,30 | 0,30 |
| Natriumsulfit | -- | 1,00 | -- | 1,00 | -- | 1,00 | -- | 1,00 | -- | 1,00 |
| erzielte Färbungen | Marineblau | | Blaugrün | | Gelbtürkis | | Rotbraun | | Blaugrün | |

In allen Fällen wurden glänzende, kräftige Färbungen erhalten, wobei überraschenderweise die erfindungsgemäßen Zusammensetzungen Nr. 13, 15, 17, 19 und 21 durchgängig eine stärkere Farbintensität sowie einen ausgeprägteren Glanz gegenüber den Natriumsulfit enthaltenden Zusammensetzungen aufwiesen.
Auch erfolgte der Farbaufzug schneller (bereits nach etwa 15 Minuten) als mit den Zusammensetzungen, die ein Reduktionsmittel enthielten.
Nach dreimonatiger Lagerung bei 40°C traten bei den genannten Beispielen keinerlei Veränderungen auf, während die Zusammensetzungen nach den Beispielen 14, 16, 18, 20 und 22 für die Haarfärbung nicht mehr geeignet waren.

## Patentansprüche

1. Wäßrige Haarfärbemittelzusammensetzung auf Basis eines Oxidationsfarbstoffvorprodukt-Systems, enthaltend
a) mindestens ein Tetraaminopyrimidin und/oder Triaminohydroxypyrimidin bzw. deren wasserlösliche Salze;
b) mindestens eine Kupplersubstanz, ausgewählt aus Resorcin, 2-Methylresorcin, 4-Chlorresorcin, 2-Aminophenol, 3-Amino-phenol, 5-Amino-2-methylphenol, 6-Amino-2-methylphenol, α-Naphthol, 1,3-Diaminobenzol, 2-Amino-4-hydroxyethylaminoanisol und/oder 2,4-Diaminophenoxyethanol bzw. deren Salzen; und
c) mindestens einen direktziehenden kationischen Farbstoff
**dadurch gekennzeichnet, daß** die Zusammensetzung kein Reduktionsmittel enthält.

2. Haarfärbemittel nach Anspruch 1, enthaltend als Entwicklersubstanz 2,4,5,6-Tetraaminopyrimidin und/oder ein wasserlösliches Salz desselben.

3. Haarfärbemittel nach Anspruch 1, enthaltend 4-Hydroxy-2,5,6-triaminopyrimidin und/oder ein wasserlösliches Salz desselben.

4. Verfahren zum Färben von menschlichen Haaren, wobei eine wäßrige Oxidationsfarbstoffvorprodukt-Zusammensetzung, enthaltend
a) mindestens ein Tetraaminopyrimidin und/oder Triaminohydroxypyrimidin bzw. deren wasserlösliche Salze;
b) mindestens eine Kupplersubstanz, ausgewählt aus Resorcin, 2-Methylresorcin, 4-Chlorresorcin, 2-Aminophenol, 3-Amino-phenol, 5-Amino-2-methylphenol, 6-Amino-2-methylphenol, α-Naphthol, 1,3-Diaminobenzol, 2-Amino-4-hydroxyethylaminoanisol und/oder 2,4-Diaminophenoxyethanol bzw. deren Salzen; und
c) mindestens einen direktziehenden kationischen Farbstoff
die frei von Reduktionsmitteln ist, mit einer wäßrigen Peroxid-Zusammensetzung gemischt, auf das Haar aufgebracht und nach erfolgter Einwirkung aus dem Haar ausgespült wird.

## Claims

1. Aqueous hair dyeing composition on the basis of an oxidation dyestuff precursor system, comprising
a) at least one tetraaminopyrimidine and/or triaminohydroxy pyrimidine or the water-soluble salt thereof;
b) at least one coupling substance, selected from resorcinol, 2-methyl resorcinol, 4-chlororesorcinol, 2-aminophenol, 3-aminophenol, 5-amino-2-methyl phenol, 6-amino-2-methyl phenol, α-naphthol, 1,3-diaminobenzene, 2-amino-4-hydroxy ethyl aminoanisol and/or 2,4-diamino-phenoxyethanol or the salts thereof and
c) at least one direct-acting cationic dyestuff
**characterized in that** the composition comprises no reducing agent.

2. Hair dyeing composition according to claim 1, comprising as developing substance 2,4,5,6-tetraaminopyrimidine and/or a water-soluble salt thereof.

3. Hair dyeing composition according to claim 1, comprising 4-hydroxy-2,5,6-triamino pyrimidine and/or a water-soluble salt thereof.

4. Process for the dyeing of human hair, wherein an aqueous oxidation dyestuff precursor composition, comprising
a) at least one tetraaminopyrimidine and/or triaminohydroxy pyrimidine or the water-soluble salts thereof;
b) at least one coupling substance, selected from 2selected from resorcinol, 2-methyl resorcinol, 4-chlororesorcinol, 2-aminophenol, 3-aminophenol, 5-amino-2-methyl phenol, 6-amino-2-methyl phenol, α-naphthol, 1,3-diaminobenzene, 2-amino-4-hydroxy ethyl aminoanisol and/or 2,4-diamino-phenoxyethanol or the salts thereof and
c) at least one direct-acting cationic dyestuff which is free from reducing agents, is mixed with an aqueous peroxide composition, is applied onto the hair and rinsed therefrom after completed processing.

## Revendications

1. Composition de teinture capillaire aqueuse à base d'un système de produit précurseur de colorant d'oxydation, contenant
a) au moins une tétraaminopyrimidine et/ou une triaminohydroxypyrimidine, ou leurs sels hydrosolubles;
b) au moins un agent de couplage, choisi parmi la résorcine, la 2-méthylrésorcine, la 4-chlororésorcine, le 2-aminophénol, le 3-aminophénol, le 5-amino-2-méthylphénol, le 6-amino-2-méthylphénol, l'α-naphtol, le 1,3-diaminobenzène, le 2-amino-4-hydroxyéthylaminoanisole et/ou le 2,4-diaminophénoxyéthanol ou leurs sels; et
c) au moins un colorant direct cationique
**caractérisée en ce que** la composition ne contient aucun agent réducteur.

2. Teinture capillaire selon la revendication 1, contenant en tant que substance révélatrice, la 2,4,5,6-tétraaminopyrimidine et/ou un sel hydrosoluble de cette dernière.

3. Teinture capillaire selon la revendication 1, contenant de la 4-hydroxy-2,5,6-triaminopyrimidine et/ou un sel hydrosoluble de cette dernière.

4. Procédé de teinture des cheveux humains, dans lequel une composition aqueuse de produit précurseur de colorant d'oxydation, contenant
a) au moins une tétraaminopyrimidine et/ou une triaminohydroxypyrimidine, ou leurs sels hydrosolubles;
b) au moins un agent de couplage, choisi parmi la résorcine, la 2-méthylrésorcine, la 4-chlororésorcine, le 2-aminophénol, le 3-aminophénol, le 5-amino-2-méthylphénol, le 6-amino-2-méthylphénol, l'α-naphtol, le 1,3-diaminobenzène, le 2-amino-4-hydroxyéthylaminoanisole et/ou le 2,4-diaminophénoxyéthanol ou leurs sels; et
c) au moins un colorant direct cationique,
qui est exempte d'agents réducteurs, est mélangée avec une composition aqueuse de peroxyde, appliquée sur le cheveu et éliminée du cheveu par rinçage après que l'effet ait eu lieu.
